# EUROPÄISCHE PATENTSCHRIFT

(11) **EP 3 799 599 B1**
(45) Veröffentlichungstag und Bekanntmachung des Hinweises auf die Patenterteilung: **06.04.2022**
(21) Anmeldenummer: 19758329.7
(22) Anmeldetag: 18.07.2019
(51) Int. Cl.: C12M 1/107, B01D 53/58, C02F 1/20

(54) **VORRICHTUNG UND VERFAHREN ZUR NEUTRALISIERUNG EINER AMMONIUMSULFATLÖSUNG**
DEVICE AND METHOD FOR NEUTRALIZING AN AMMONIUM SULPHATE SOLUTION
DISPOSITIF ET PROCÉDÉ DE NEUTRALISATION D'UNE SOLUTION DE SULFATE D'AMMONIUM

(30) Priorität: 24.08.2018 DE 102018120720
(43) Veröffentlichungstag der Anmeldung: 07.04.2021
(73) Patentinhaber: Biogastechnik Süd GmbH, 88316 Isny im Allgäu (DE)
(72) Erfinder: MAIER, Clemens, 88316 Isny-Sommersbach (DE); MAIER, Gregor, 88316 Isny-Sommersbach (DE)
(74) Vertreter: Höchtl, Walter
(86) Internationale Anmeldenummer: PCT/DE2019/100666
(87) Internationale Veröffentlichungsnummer: WO 2020/038518

(56) Entgegenhaltungen:
- EP-A1- 1 318 105
- EP-A1- 2 808 304

## Beschreibung

Die Erfindung bezieht sich auf eine Vorrichtung und ein Verfahren zur Neutralisierung einer Ammoniumsulfatlösung (ASL), die bei der Verdampfung von organischen Substraten und dem anschließenden Waschen des Brüden in sogenannten Brüdenwäschern entsteht.

Organische Substrate werden auch als Biomasse bezeichnet - wie beispielsweise Klärschlamm oder Biomasse aus nachwachsenden Rohstoffen (NaWaRo). Die organischen Substrate können aus einem Lager für die Biomasse, einer sogenannten Vorgrube, einem Vorgärer, einem Fermenter, z. Bsp. aus einem Biogas - Fermenter stammen; oder es kann sich z. Bsp. um Gülle aus einem landwirtschaftlichen Betrieb oder pumpfähige Biomasse handeln. Prinzipiell kann jedes organische Substrat in Fermentern oder anderen Aufschlussmodulen aufgeschlossen werden. Der hohe Anteil an Stickstoff in diesen Substraten wird in den sogenannten Verdampfern, wie sie beispielsweise in Biogasanlagen zum Einsatz kommen, zu giftigem Ammoniak (NH₃ H₂O) umgewandelt.

Aus der DE 10 2009 036 874 A1 ist eine Vorrichtung bzw. ein Verfahren zur energetisch optimierten Gewinnung von Ammoniumsalzen aus Ammoniak oder Ammoniumverbindungen enthaltenden Flüssigkeiten, wie Gülle, Abläufe von Biogasanlagen etc., bekannt, wobei die flüssige Phase einem Desorber-Stripp-Prozess unterzogen wird, wobei in der Stripp-Kolonne im Gegenstrom Brüden geführt werden, wobei der hierdurch mit Ammoniak angereicherte Brüdenstrom mit einer wässrigen, Säuren und/oder Salze enthaltenden Lösung, insbesondere einer schwefelsauren Ammoniumsulfatlösung in einer Absorptionsstufe gewaschen wird, um Ammoniak vollständig in Ammoniumsalz umzusetzen und wobei der die Absorptionsstufe verlassende Brüdenstrom in den Desorber, einen Kreislauf bildend, zurückgeführt und dort als Desorptionsmedium genutzt wird.

Aus der europäischen Patentanmeldung EP 2 808 304 A1 ist es unter Verweis auf den Stand der Technik bekannt, dass wässrige, organische Rückstände aus anaeroben Prozessen, die Ammoniak und/oder Ammonium Ionen enthalten, durch Verdampfen eingedickt werden. Diese Rückstände stammen beispielsweise aus der Biogaserzeugung, aus Faultürmen von Kläranlagen oder aus Tierhaltungen. Häufig werden derartige Rückstände auch als Gärreste bezeichnet. Durch das Verdampfen entsteht ein Konzentrat, das entsprechend auch als Rückstands- oder Gärrestekonzentrat bezeichnet wird, welches als Wirtschaftsdünger einsetzbar ist. Als Dampfphase entsteht ein mit Ammoniak beladener Brüden, der im Wesentlichen aus Wasserdampf und gasförmigen Ammoniak besteht.

Dieser Brüden aus Wasserdampf und gasförmigen Ammoniak wird in sogenannten Brüdenwäschern durch Zugabe von Säure, in der Regel Schwefelsäure, "gewaschen". Dabei reagiert das basische Ammoniak mit der Säure zu einer Ammoniumsulfatlösung. Der aus dem Brüdenwäscher austretende Wasserdampf sollte möglichst frei von Ammoniak sein, damit er - nach Kondensierung - ins Abwasser geleitet werden kann. Dies wird dadurch erreicht, dass durch die "Waschung" eine Ammoniaksulfatlösung im niedrigen pH Bereich, von beispielsweise pH=3 erzielt wird. Je niedriger der pH-Wert umso reiner ist das Kondensat.

Die gewonnene Ammoniumsulfatlösung mit einem pH-Wert von ca. pH = 3 hat allerdings den Nachteil, dass bei einem pH-Wert unter 4 ein Transport sehr schwierig ist, weil sie als Gefahrgut eingestuft wird, sämtliche Dichtungsmaterialen auf Dauer Probleme bereiten, und vor allem das ASL beim Ausbringen stark kalkzehrend ist und die Pflanzen bei der Ausbringung verätzt werden können. Deshalb sollte der pH-Wert vor der Ausbringung angehoben werden.

Aus der deutschen Patentanmeldung DE 10 2010 017 334 A1 ist ein Verfahren zur Behandlung von wässrigen, organischen, Ammoniak und/oder Ammonium enthaltenden Rückständen aus anaeroben Prozessen sowie eine Anlage zur Durchführung des Verfahrens bekannt. Der aus dem Verdampfer stammende, ammoniakhaltige Brüden wird zunächst kondensiert und anschließend so weiterbehandelt, dass das Ammoniak aus dem Kondensat durch Strippen entfernt wird. Zur Anpassung des pH-Werts für den Strippvorgang wird Lauge, z. B. Natronlauge, benötigt. Abschließend muss der pH-Wert für die Einleitung in Gewässer wieder neutralisiert werden, wofür erneut Chemikalien einzusetzen sind.

Aufgabe der Erfindung ist es deshalb, eine Vorrichtung und ein Verfahren zur Verfügung zu stellen, mit dem das aus den Verdampfungsprozessen von organischen Rückständen gewonnene Ammoniumsulfatlösung bereits beim Waschen des Brüden neutralisiert wird.

Ein Ausführungsbeispiel der Erfindung wird nachfolgend anhand der Figur beschrieben.

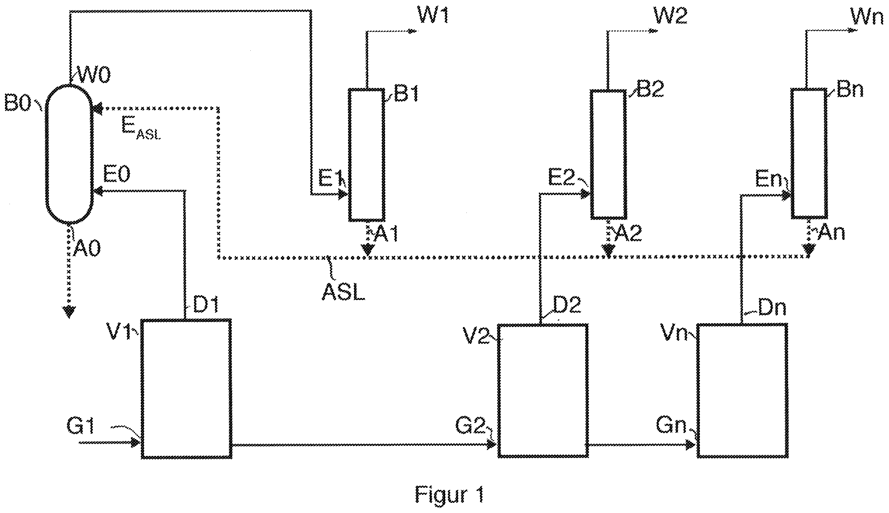

Die Figur 1 zeigt eine Vorrichtung zur Neutralisierung einer Ammoniumsulfatlösung mit Verdampfern V1 bis Vn und Brüdenwäschern B1 bis Bn. Dem ersten Brüdenwäscher B1 ist ein sogenannter neutralisierender Brüdenwäscher B0 vorgeordnet. Den Verdampfern V2 bis Vn ist jeweils ein Brüdenwäscher B2 bis Bn zugeordnet, während dem ersten Verdampfer V1 der neutralisierende Brüdenwäscher B0 zugeordnet ist.

Den Verdampfern V1 bis Vn wird ammoniakhaltiger Rückstand, beispielsweise aus Biogasanlagen, Kläranlagen oder aus der Tierhaltung, beispielsweise als Gärrest über die Eingänge G1 bis Gn zugeführt. Soweit es sich um mehrstufige Verdampfungsanlagen handelt, wird dem Verdampfer Vn der Gärrest aus dem vorgeordneten Verdampfer Vn-1 zugeführt. Die Verdampfer V1 bis Vn können aber auch aus unterschiedlichen Quellen gespeist werden.

Der in den Verdampfern V1 bis Vn entstehende Brüden wird den Brüdenwäschern B0, B2 bis Bn über die Auslässe D1 bis Dn und die Einlässe E0, E2 bis En zugeführt. Dem Brüdenwäscher B1 wird der teilgereinigte Brüden über den Auslass W0 und den Einlass Elzugeführt.

Die in den Brüdenwäschern B1 bis Bn gewonnene Ammoniumsulfatlösung wird über deren ASL-Auslässe A1 bis An und über die gemeinsame Leitung ASL zum neutralisierenden Brüdenwäscher B0 zurückgeführt. Der gereinigte Brüden (Wasserdampf) aus den Brüdenwäschern B1 bis Bn verlässt diese über die Leitungen W1 bis Wn und kann nach Kondensierung ins Abwasser eingeleitet werden. Dazu kann der gereinigte Brüden beispielsweise auch zentral auf bekannte Weise mittels einer Vakuumpumpe abgesaugt werden. Die bei der Kondensierung freiwerdende Wärme kann - wie aus dem Stand der Technik hinreichend bekannt - zur Beheizung der Verdampfer V2 bis Vn genutzt werden.

Die Brüden aus den Verdampfern V1 bis Vn weisen einen Ammoniakgehalt mit einem pH-Wert von 6 - 8 auf, also nahezu im neutralen Bereich. Würde der Brüden mit diesem pH-Wert gewaschen, wäre der entstehende "Wasserdampf" noch stark Ammoniak beladen und könnte nicht ins Abwasser eingeleitet werden. Deshalb wird der Brüden in den Brüdenwäschern B1 bis Bn solange gewaschen, bis die über die Leitungen ASL austretende Ammoniumsulfatlösung einen pH-Wert von pH = ungefähr 3, insbesondere zwischen 2,2 und 3,2 aufweist. - Die pH-Werte können auf dem Fachmann bekannte Weise mit einer pH-Sonde gemessen werden. Auf eine Darstellung solcher Sonden wurde deshalb der Übersichtlichkeit halber verzichtet.

Bei einem pH-Wert zwischen 2,2 und 3,2 ist der gereinigte Dampf sauber genug, um nach Kondensierung ins Abwasser geleitet zu werden. Andrerseits ist die Ammoniumsulfatlösung noch relativ sauer, das heißt schwefelsäurehaltig, so dass die eingangs beschriebenen Nachteile entstehen würden.

Deshalb wird die Ammoniumsulfatlösung aus den Brüdenwäschern B1 bis Bn zum Brüdenwäscher B0 zurückgeführt und dort auf einen pH-Wert von pH = annähernd 7, also in den neutralen Bereich angehoben. Zur Anhebung des pH Wertes muss jedoch keine Lauge zugeführt werden, sondern es wird der Brüden aus dem Verdampfer V1 dazu genutzt.

Der Brüden aus dem Verdampfer V1 gelangt über den Auslass D1 in den neutralisierenden Brüdenwäscher B0. Da der Brüden einen pH-Wert von pH =6 bis pH = 8 aufweist, kann die im Brüdenwäscher B0 gesammelte Ammoniumsulfatlösung mit diesem Brüden auf einen pH-Wert von pH >= 6 angehoben werden. Dazu wird die Ammoniumsulfatlösung solange im neutralisierenden Brüdenwäscher B0 im Kreis gepumpt, bis sie den gewünschten pH-Wert erreicht hat, dann wird sie ausgeschleust, anschließend kommt wieder neue Ammoniumsulfatlösung über die Leitung ASL von den Brüdenwäschern B1 bis Bn in den neutralisierenden Brüdenwäscher B0 und der Vorgang wiederholt sich.

Die so neutralisierte Ammoniumsulfatlösung wird dann über die Leitung A0 ausgeschleust.

## Patentansprüche

1. Vorrichtung zur Neutralisierung einer Ammoniumsulfatlösung mit mindestens einem Verdampfer (V1-Vn) mindestens einem Brüdenwäscher (B1-Bn), mit einem Brüdeneinlass (E1-En), einem ASL-Auslass (A1-An) für die gewaschene Ammoniumsulfatlösung, sowie einem Dampfauslass (W1-Wn) für den gewaschenen Brüden,
**dadurch gekennzeichnet, dass**
ein neutralisierender Brüdenwäscher (B0) vorgesehen ist, mit einem ASL-Einlass (EASL), einem Dampfauslass (W0), einem Brüdeneinlass (E0) und einem ASL-Auslass (A0), wobei der ASL-Auslass (A1-An) des mindestens einen Brüdenwäschers (B1-Bn) mit dem Einlass (EASL) des neutralisierender Brüdenwäschers (B0) und dessen Brüdeneinlass (E0) für den ammoniakhaltigen Brüden mit dem Brüdenauslass (D1) des Verdampfers (V1) verbunden ist, und wobei der Dampfauslass (W0) des neutralisierenden Brüdenwäschers (B0) mit dem Dampfeinlass (E1) des Brüdenwäschers (B1) verbunden ist.

2. Vorrichtung nach Anspruch 1, wobei die ASL-Auslässe (A2 bis An) aus weiteren Brüdenwäschern (B2 bis Bn), mit dem ASL-Einlass (EASL) des neutralisierenden Brüdenwäschers (B0) verbunden sind.

3. Verfahren zur Neutralisierung einer Ammoniumsulfatlösung mit mindestens einem Verdampfer (V1-Vn)
mindestens einem Brüdenwäscher (B1-Bn), die jeweils einen ASL Auslass (A1-An) für die Ammoniumsulfatlösung aufweisen,
wobei der Brüden aus dem mindestens einem Verdampfer (V1-Vn) in dem mindestens einen Brüdenwäscher (B1-Bn) solange gewaschen wird, bis die über die ASL Auslässe (A1-An) austretende Ammoniumsulfatlösung einen pH-Wert von ungefähr gleich 3, insbesondere zwischen 2,2 und 3,2 aufweist,
**dadurch gekennzeichnet, dass**
ein neutralisierender Brüdenwäscher (B0) vorgesehen ist, in den die in dem mindestens einen Brüdenwäscher (B1-Bn) gewonnene Ammoniumsulfatlösung zurückgespeist wird und dort mit dem Brüden aus dem ersten Verdampfer (V1) solange gewaschen wird, bis sich ein pH-Wert der Ammoniumsulfatlösung im neutralen Bereich ergibt.

4. Verfahren nach Anspruch 3, **dadurch gekennzeichnet, dass** die aus weiteren Brüdenwäschern (B2 bis Bn) gewonnene Ammoniumsulfatlösung in den neutralisierenden Brüdenwäscher (B0) eingespeist wird.

## Claims

1. Apparatus for the neutralisation of an ammonium sulphate solution having
at least one evaporator (V1-Vn)
at least one vapour scrubber (B1-Bn), having a vapour inlet (El-En), an ASL outlet (A1-An) for the scrubbed ammonium sulphate solution, and a vapour outlet (W1-Wn) for the scrubbed vapour,
**characterised in that**
a neutralising vapour scrubber (B0) is provided, having an ASL inlet (EASL), a vapour outlet (W0), a vapour inlet (E0) and an ASL outlet (A0), wherein the ASL outlet (A1-An)of the at least one vapour scrubber (B1-Bn) is connected to the inlet (EASL) of the neutralising vapour scrubber (B0) and its vapour inlet (E0) for the vapour containing ammonia is connected to the vapour outlet (D1) of the evaporator (V1), and wherein the vapour outlet (W0) of the neutralising vapour scrubber (B0) is connected to the vapour inlet (El) of the vapour scrubber (Bl).

2. The apparatus according to claim 1, wherein the ASL outlets (A2 to An) from further vapour scrubbers (B2 to Bn), are connected to the ASL inlet (EASL) of the neutralising vapour scrubber (B0).

3. Process for the neutralisation of an ammonium sulphate solution comprising at least one evaporator (V1-Vn)
at least one vapour scrubber (B1-Bn), each having an ASL outlet (A1-An)for the ammonium sulphate solution,
wherein the vapour from the at least one evaporator (V1-Vn) is washed in the at least one vapour scrubber (B1-Bn) until the ammonium sulphate solution leaving via the ASL outlets (A1-An)has a pH value of approximately equal to 3, in particular between 2.2 and 3.2,
**characterised in that**
a neutralising vapour scrubber (B0) is provided, into which the ammonium sulphate solution obtained in the at least one vapour scrubber (B 1-Bn) is fed back and is washed there with the vapour from the first evaporator (V1) until a pH value of the ammonium sulphate solution is obtained in the neutral range.

4. The process according to claim 3, **characterised in that** the ammonium sulphate solution obtained from further vapour scrubbers (B2 to Bn) is fed into the neutralising vapour scrubber (B0).

## Revendications

1. Dispositif de neutralisation d'une solution de sulfate d'ammonium comprenant
au moins un évaporateur (V1-Vn)
au moins un laveur de buées (B1-Bn), avec une entrée de buées (El-En), une sortie d'ASL (A1-An)pour la solution de sulfate d'ammonium lavée, ainsi qu'une sortie de vapeur (W1-Wn) pour les buées lavées,
**caractérisé en ce que**
un laveur de buées neutralisant (B0) est prévu, avec une entrée d'ASL (EASL), une sortie de vapeur (W0), une entrée de buées (E0) et une sortie d'ASL (A0), la sortie ASL (A1-An)de l'au moins un laveur de vapeur (B1-Bn) étant reliée à l'entrée (EASL) du laveur de vapeur neutralisant (B0) et son entrée de vapeur (E0) pour la vapeur contenant de l'ammoniac étant reliée à la sortie de vapeur (D1) de l'évaporateur (V1), et dans lequel la sortie de vapeur (W0) de l'épurateur de vapeurs neutralisantes (B0) est reliée à l'entrée de vapeur (E1) de l'épurateur de vapeurs (B1).

2. Dispositif selon la revendication 1, dans lequel les sorties ASL (A2 à An) d'autres laveurs de vapeur (B2 à Bn) sont reliées à l'entrée ASL (EASL) du laveur de vapeur neutralisant (B0).

3. Procédé de neutralisation d'une solution de sulfate d'ammonium
comprenant au moins un évaporateur (V1-Vn)
au moins un laveur de buées (B1-Bn), qui présentent chacun une sortie ASL (A1-An)pour la solution de sulfate d'ammonium,
les buées provenant de l'au moins un évaporateur (V1-Vn) étant lavées dans l'au moins un laveur de buées (B1-Bn) jusqu'à ce que la solution de sulfate d'ammonium sortant par les sorties ASL (A1-An)présente un pH approximativement égal à 3, en particulier entre 2,2 et 3,2,
**caractérisé en ce que**
il est prévu un laveur de buées neutralisant (B0) dans lequel la solution de sulfate d'ammonium obtenue dans le au moins un laveur de buées (B1-Bn) est réintroduite et y est lavée avec les buées provenant du premier évaporateur (V1) jusqu'à ce que l'on obtienne une valeur de pH de la solution de sulfate d'ammonium dans la plage neutre.

4. Procédé selon la revendication 3, **caractérisé en ce que** la solution de sulfate d'ammonium obtenue à partir d'autres laveurs de buées (B2 à Bn) est introduite dans le laveur de buées neutralisant (B0).
